# EUROPEAN PATENT SPECIFICATION

(11) **EP 4 377 439 B1**
(45) Date of publication and mention of the grant of the patent: **19.02.2025**
(21) Application number: 22748493.8
(22) Date of filing: 29.07.2022
(51) Int. Cl.: C12M 3/00, A61L 27/38, A61L 29/14, C12M 1/12, C12M 1/26, D01F 9/04

(54) **PREPARATION OF CELL-CONTAINING HYDROGEL**
HERSTELLUNG EINES ZELLHALTIGEN HYDROGELS
PRÉPARATION D'HYDROGEL CONTENANT DES CELLULES

(30) Priority: 30.07.2021 NL 2028890
(43) Date of publication of application: 05.06.2024
(73) Proprietor: Mosa Meat B.V., 6229 PM Maastricht (NL)
(72) Inventor: BREEMHAAR, Jonathan Jan, 6229 PM Maastricht (NL); MAZARI, Nik, 6229 PM Maastricht (NL); MELZENER, Lea, 6229 PM Maastricht (NL); SONNECK, Ludwig Jakob, 6229 PM Maastricht (NL); SPAANS, Sergio, 6229 PM Maastricht (NL); WIELAND, Eric Wolfgang, 6229 PM Maastricht (NL); VAN ZOMEREN, Nick, 6229 PM Maastricht (NL); HAUCK, Nicolas Wolfgang, 6229 PM Maastricht (NL)
(74) Representative: Moura, Miguel
(86) International application number: PCT/NL2022/050452
(87) International publication number: WO 2023/009007

(56) References cited:
- WO-A1-2020/137304
- CN-A- 112 999 425
- US-A1- 2020 024 560
- ZIDARIC TANJA ET AL: "Cultured Meat: Meat Industry Hand in Hand with Biomedical Production Methods", FOOD ENGINEERING REVIEWS, SPRINGER, US, vol. 12, no. 4, 26 August 2020 (2020-08-26), pages 498 - 519, XP037289819, ISSN: 1866-7910, [retrieved on 20200826], DOI: 10.1007/S12393-020-09253-W

## Description

### TECHNICAL FIELD

The aspects and embodiments thereof relate to the field of cultured meat, in particular devices and method for preparation of cell-containing hydrogel.

### BACKGROUND

Since ancient times, meat has been a major source of high-quality protein in the human diet, and to this day it continues to provide nutrition to the exponentially growing population of the world. However, meat is a very inefficient source of food and its production has increased so much that it is now one of the largest contributors to a number of serious problems such as animal welfare, pollution and food safety issues.

The ideal replacement for animal meat would be meat produced through tissue engineering. Virtually all aforementioned downsides of meat production would be eradicated but the consumers could still enjoy the meat.

Growing muscle tissue has been subject to research & development for a long time in the medical and research field, and conducted by artificially proliferating two-dimensional (2D) muscle cells into three-dimensional (3D) tissue-specific progenitors in the presence of growth and differentiation media.

GB2573327B discloses an apparatus for the production of tissue from cells. The apparatus comprises an elongate body and at least one trough; the elongate body having at least one circumferential groove and being operable to extend, by close-fitting relationship, centrally through the at least one trough. When using the device of GB2573327B, cells and a liquid hydrogel, comprising a scaffolding biomaterial, are added into at least one trough of the apparatus. The scaffolding biomaterial is subsequently cross-linked by exposure to heat or ultraviolet radiation, and as such a gelated transitioning intermediate is obtained with a shape corresponding to the shape of the at least one trough.

US2020024560A1 discloses a bioreactor and methods for using said bioreactor for drug screening. The bioreactor can include a microfibrous hydrogel scaffold, that can be made of a composite alginate-gelatin methacryloyl (GeIMA) bioink, and that can have endothelial cells directly embedded within the scaffold. The scaffold can further be seeded with cardiomyocytes so that said bioreactor has a controlled anisotropy, and the scaffold can be placed in a chamber defined by a PDMS half pieces, that compress the scaffold slightly when the PDMS half pieces are fastened to each other.

### SUMMARY

It is preferred to at least partially automate the process of obtaining a cell-containing hydrogel and/or increase the speed with which the cell-containing hydrogel can be obtained. Present methods and devices only allow for very small quantities - in the order of grams - of meat to be produced, for example due to limitations in the amount of cell-containing hydrogel which can be produced. It is desired to be able to produce larger quantities of meat in relatively less time and/or with relatively less resources and/or with relatively less human effort.

It is found that the presently used cross-linking process using heat is relatively slow. Crosslinking based on ultraviolet radiation, while being faster, typically requires the use of compounds that are not considered safe for foodstuff such as cultured meat. Cross-linking processes based on for instance ionic bonds or quick chemical reactions may be faster than cross-linking processes using heat and safer than ultraviolet radiation. Cross-linking based on ionic bonds may for instance be achieved by contacting a first liquid comprising the polymer chains with a second liquid comprising ionic moieties.

It has been observed that a faster cross-linking process than e.g. thermal or ultraviolet radiation crosslinking, when used with the device of GB2573327B, posed challenges. For instance, ionic crosslinking results in waves due to the two low viscosity liquids mixing, and subsequently solidifying in this shape. As such, it proved difficult to obtain a homogeneous shape and thickness of the gelated transitioning intermediate.

A first aspect provides a method of preparing a cell-containing hydrogel for use in the production of cultured meat. The method comprising the steps of positioning a central body inside a circumferential member, thereby forming an annular volume between the central body and an inner wall of the circumferential member, filling the annular volume with a cell-containing fluid, moving the central body relative to the circumferential member such that the central body moves through an outlet opening of the circumferential member, thereby creating an access area between the central body and the circumferential member, and exposing the access area to a cross-linking fluid to allow crosslinking of the cell-containing fluid to obtain a cell-containing hydrogel.. The method allows the use of a fast cross-linking process by mixing the fluid cell culture with the cross-linking fluid. Different steps in the method may be performed at least partially simultaneously.

A hydrogel is a network wherein the discontinuous phase is solid and the continuous phase is water. The discontinuous phase is typically a network of hydrophilic polymer chains, which are crosslinked to form a three-dimensional network. Gels may be considered semi-solids and typically exhibit little to no flow at steady-state. The structural integrity of the hydrogel is typically not compromised by the presence of water. Hydrogels may for instance be capable of absorbing water to a high extent. Crosslinking (also referred to as gelation) of the polymer chains may be physical or chemical. Physical crosslinks for instance include ionic interactions, chain entanglement and hydrogen bonds. Chemical crosslinks are typically based upon covalent bonds between polymer chains.

The cell-containing fluid typically comprises cells of non-human mammal origin. These cells may for instance be myosatellite cells. Typically, the cells are proliferated and ready to fuse into myotubes and long muscle fibres.

The cell-containing fluid further typically comprises a polysaccharide. Such polysaccharides may for instance include starch, chitin and alginate. Preferably the cell-containing fluid comprises alginate. Alginate is an anionic biopolymer comprising α-L-guluronate (also referred to as G) and β-D-mannuronate (also referred to as M). Alginates are often used in bioengineering due to its biocompatibility and non-toxicity. Further, alginate may encourage cell proliferation and mammalian cells typically do not express any enzymes that may degrade the polysaccharide.

In particular, an alginate with a specific molecular weight and/or specific composition may be preferred. A suitable alginate for instance has a low molecular weight, such as between 10 to 50 kDa. A suitable composition may be a M/G ratio of 0.8 to 1.5. This allows for increased biodegradability and faster stress relaxation. The faster stress relaxation may allow for the cells to spread within a hydrogel based on such alginates. Further, the faster stress relaxation typically allows for cell-cell contacts and myotubes to form. Additionally, the specific molecular weight and/or specific composition may allow for an optimal stiffness for cellular alignment.

The polysaccharide is preferably conjugated with one or more cell-adhesion peptides. The cell-adhesion peptide may be capable of binding to a receptor on the cell to encourage several processes, such as cell migration, spreading, guidance and differentiation. Cell adhesion peptides may attach to various integrin receptors on the cell surface. Accordingly, the cell-adhesion peptide may be an integrin-binding ligand, such as the peptide motif RGD (Arginine-Glycine-Aspartic acid). Additionally, the polysaccharide may be conjugated with a plurality of different cell-adhesion peptides.

Preferably, the cell-containing fluid comprises an aqueous solution of an alginate, typically sodium alginate, that is conjugated to one or more cell-adhesion peptides and a cell mixture comprising cells of non-human mammal origin.

The cell-containing fluid is typically exposed to the cross-linking fluid to allow for crosslinking of the cell-containing fluid. The cell-containing fluid and the crosslinking fluid are typically low viscous liquids, for instance the viscosity may resemble the viscosity of water at ambient temperature (*i.e.* 20°C). The cross-linking fluid is accordingly what provides the required conditions and/or components to allow for crosslinking. Crosslinking may result in the formation of a cell-containing hydrogel.

The fast crosslinking process used in the present invention is typically sufficiently fast to allow for quick or preferably instant crosslinking of the polymer chains. Typically, for the fast crosslinking process, the cross-linking fluid first contacts the outer surface of the cell-containing fluid. Accordingly, the outer layer of the cell-containing fluid may instantaneously form a gel thereby preventing the cell-containing fluid to spread out or lose its shape as defined by the moulding volume. The inner core of the cell-containing fluid surrounding the central body may require some additional time for crosslinking. Therefore, the crosslinking time is typically between 1-10 minutes, preferably between 3-6 minutes. This may allow for roughly all the cell-containing fluid to form the cell-containing hydrogel.

Instant crosslinking may imply that the crosslinking process is sufficiently fast to fully or at least partially prevent the cell-containing fluid from flowing off the central body after being exposed to the crosslinking fluid.

A particularly suitable alginate-based hydrogel with an optimal environment for the production of muscle tissue is described in PCT/NL2021/050068 (filed by the present applicant, not yet published).

Dependent on the type of crosslinking and type of polysaccharide, the crosslinking fluid may comprise divalent cations, a covalent crosslinker, and/or a buffer. A suitable covalent crosslinker may be genipin. For instance, when using alginate as polysaccharide, divalent cations such as calcium ions or magnesium ions (Mg²⁺) are preferred. Preferably calcium (Ca²⁺) ions are used. The divalent cations may be present in the cross-linking fluid as a dissociated salt. The cross-linking fluid may accordingly comprise an aqueous solution of a salt, such as calcium chloride (CaCl₂) or magnesium chloride (MgCl₂).

Preferably, the cross-linking fluid comprises an aqueous solution of calcium chloride, as this typically results in fast gelation of the cell-containing fluid. Typically, the concentration of the divalent cations is between 0.05 and 0.5M. The preferred concentration of the cations and accordingly the degree of crosslinking may present suitable chemical, topographical and mechanical properties for myosatellite cells to migrate, spread, align and differentiate into muscle tissue.

The buffer may for instance be used to provide an optimal pH or at least a pH within a range that is sufficient for self-crosslinking. Self-crosslinking is herein used to describe the process that the polymeric chains of the polysaccharide may form the polymeric network without being spaced by *e.g.* calcium ions or a crosslinker.

The polysaccharide may have functional groups capable of reacting with a covalent crosslinker. The functional groups may be inherently present in the polysaccharide or may be synthetically introduced. Covalent crosslinkers are typically compounds that comprise reactive groups, for instance reactive end-groups. These reactive groups may react with the functional groups of the polysaccharide, thereby forming covalent bonds that result in the crosslinking of the polymer chains. In such cases, it may be preferred that the cross-linking fluid comprises a buffer. Such buffers may for instance have a suitable pH at which the crosslinker and polysaccharide react. The crosslinker may be present in the cell-containing fluid and/or in the crosslinking-fluid. The cross-linking fluid accordingly provides the environment for the cell-containing fluid to be subjected to fast gelation.

In some cases, particularly when the used polysaccharide is alginate, the cell-containing fluid may be at least partially gelated (*i.e.* the viscosity is increased but the cell-containing hydrogel is not yet formed) by a slow gelation or internal gelation. Preferably, the cell-containing fluid is at least partially gelated in the mould. Advantageously, this may allow for the cell-containing fluid to obtain a high enough viscosity that minimizes any flow of the cell-containing fluid from the central body when it is exposed to the crosslinking-fluid. Accordingly, the final shape of the hydrogel is typically smoother and more defined as determined by the mould, e.g. the central body and the circumferential member. The slower gelation or internal gelation typically comprises exposing the cell-containing fluid comprising alginate to a forming fluid. This forming fluid is typically liquid, preferably it is a low viscous liquid, for instance with a viscosity similar to the viscosity of water at ambient temperature. The forming fluid may comprise calcium gluconate and/or a mixture of glucono-δ-lactone (GDL) with calcium carbonate (CaCO₃). Good results have been obtained for a forming liquid comprising a GDL/CaCO₃ mixture. The forming liquid typically slowly releases calcium ions to obtain a slower ionotropic gelation of the alginate. As the slow gelation or internal gelation is typically only partial, it is followed by exposure to the crosslinking fluid to obtain the final cell-containing hydrogel. Accordingly, the slower or internal gelation process can be used as a complementary crosslinking method to the fast crosslinking process using the crosslinking fluid.

After the cell-containing hydrogel is formed, the hydrogel may be incubated in a differentiation medium to form tissue. A differentiation medium typically comprises the components required to promote cells to differentiate. Additionally, the differentiation medium may comprise *i.a.* divalent cations to ensure that the hydrogel is not detrimentally compromised during incubation.

The cells may adhere to the cell-adhesion peptides of the polysaccharide and exert a force on the hydrogel during at least part of the method (e.g. during incubation). By exerting such forces, water may be somewhat expelled from the hydrogel resulting in the compaction of the gel. Nonetheless, the gel may still comprise water to some extent.

The method may further comprise, prior to or during the filling of at least part of the annular volume with the cell-containing fluid, moving the circumferential member over the central body.

In a further embodiment, the method comprises, after or during the filling of at least part of the annular volume with the cell-containing fluid, moving the central body through the outlet opening of the circumferential member, for example by a movement of the central body, by a movement of the circumferential member, or a movement of both.

In any method of preparing a cell-containing hydrogel for use in the production of cultured meat disclosed herein, the method may comprise a step of forming an essentially liquid-tight sealed volume for temporarily holding cell-containing fluid in the annular volume formed between the central body and the circumferential member. To form the essentially liquid-tight sealed volume, an essentially liquid-tight seal may be formed by different components of a device for use in a process for production of cultured meat.

For example, a temporary liquid-tight seal may be formed between a circumferential member and a thickened sealing section. Additionally or alternatively, a temporary liquid-tight seal may be formed between a circumferential member and a cross-linking fluid container, in particular a bottom of the cross-linking fluid container. When the temporary liquid-tight seal is formed with the cross-linking fluid container, the thickened sealing section may not be required. In embodiments, a thickened sealing section may be comprised by the cross-linking fluid container or generally formed by the cross-linking fluid container, for example by a bottom of the cross-linking fluid container.

A method of producing cultured meat is envisioned, comprising the steps of obtaining a cell-containing hydrogel using a method according to the first aspect, incubating the cell-containing hydrogel in a differentiation medium to form tissue, and producing the cultured meat from the formed tissue, which formed tissue may be muscle tissue.

A second aspect provides a device for use in a process for production of cultured meat or for use in a process for production of cell-containing hydrogel. Hence, using the device, a cell-containing hydrogel may be obtained which is cross-linked by placing the cell-containing fluid in contact with cross-linking fluid.

The device comprises a mould, comprising a central body and a circumferential member with a hollow passage therethrough, which hollow passage is provided with an inlet opening for receiving a cell-containing fluid in the hollow passage, and an outlet opening.

The device further comprises a cross-linking fluid container with a storage volume for holding a cross-linking fluid, wherein the cross-linking fluid container is arranged for accommodating at least part of the central body.

For example for performing at least part of a method according to the first aspect, the central body is arranged to be positioned at least partially inside the hollow passage of the circumferential member, thereby forming an annular volume between the central body and an inner wall of the circumferential member, the outlet opening is arranged to allow passage of at least part of the central body, and the central body and the circumferential member are arranged to be moved relative to each other in a movement in which at least part of the central body passes through the outlet opening.

A device may be suitable for production of cultured meat for example because it is sterile, and comprises or consists of materials which are suitable for use in the production of foodstuff, in particular meat, and more in particular cultured meat. Such materials may be sterilizable. Examples of materials which may be used are stainless steel, POM , nylon, 3D printable resins, silicone, any other sterilizable material, any other material which has no negative impact on cells in the cell-containing fluid, or any combination thereof.

In general, the mould is used to shape the cell-containing hydrogel. A thickened section may imply that a cross-section area of the thickened section is locally larger than a cross-section area of an adjacent part of the central body. For example, a central body may comprise a generally circular central body and a thickened sealing section with a diameter larger than that of the central body.

In general, the circumferential member with the hollow passage may be used to determine an outer shape of the cell-containing hydrogel. A circumferential member may generally resemble a hollow tube, cylinder, or pipe.

At least part of the circumferential member and/or at least part of the sealing section of the central body may be resilient or elastic, such that a shape of a first of the at least part of the circumferential member and/or at least part of the sealing section may be formed complementary to a shape of a second of the at least part of the circumferential member and/or at least part of the sealing section.

When forming muscle tissue, it may be preferred to obtain volumes of cell-containing hydrogel with a cross-sectional shape generally resembling an annulus. An annulus may be defined as a region between two closed curves, of which one curve is an inner curve contained in another one the curves - which is an outer curve. One or both of the inner and outer curve may be generally circular, ellipsoid, rectangular, or have any other shape. The inner curve may describe the shape of a through-hole in an annular volume of cell-containing hydrogel. The inner curve may be defined by the central body, and the outer curve may be defined by the circumferential member, and as such, the mould may describe the shape of the annulus.

The thickness of the hydrogel - *i.e.* the distance between the inner curve and the outer curve - may be limited in absence of blood vessels and diffusion limits.

In a first example, the device may resemble a bioreactor with a cross-linking fluid container with a storage volume for holding a cross-linking fluid. The cross-linking fluid container may remain substantially stationary in use, while the central body is moved relative to cross-linking fluid container.

In a second example, the device may resemble a pick-and-place machine, wherein the circumferential member is moved relative to a central body positioned inside a cross-linking fluid container with a storage volume for holding a cross-linking fluid.

In an embodiment of the second example, the circumferential member may be moved vertically relative to the central body. As a further option, the circumferential member may be moved horizontally relative to the central body. For example, the circumferential member may be moved using a robot arm as an actuator, or for example with three orthogonal prismatic actuators.

With the device according to the second aspect, the process of preparing a cell-containing hydrogel for use in the production of cultured meat may be at least partially automated and/or larger quantities of cell-containing hydrogel may be obtained. A control unit may be comprised by the device for the automation. Such a control unit may for example comprise an electronic processing unit for controlling one or more actuators of the device.

The device may comprise any number of pumps, valves, and conduits for transporting cell-containing fluid and/or cross-linking fluid, respectively for example into a cell-containing fluid container or annular volume, or into a cross-linking fluid container. The any number of pumps and valves may be controlled by the control unit.

The central body may be positioned in the storage volume of the cross-linking fluid container, and the circumferential member may arranged to be moved between a non-sealing position in which at least part of the central body is not surrounded by the circumferential member, and a sealing position in which an essentially liquid-tight seal is formed with the circumferential member for holding a cell-containing fluid in the annular volume between the central body and the inner wall of the circumferential member.

Embodiments of the device may further comprise an actuator for moving the circumferential member relative to the central member in a direction generally parallel to an elongation direction of the central body.

The circumferential member may be fixated relative to the cross-linking fluid container, and the central body may be arranged to be at least partially moved into the cross-linking fluid container through the hollow passage and the outlet opening of the circumferential member.

Embodiments of the device may further comprise a cell-containing fluid container for holding a volume of cell-containing fluid, and a pump for transporting cell-containing fluid into the hollow passage of the circumferential member via the inlet opening. The inlet opening may be positioned opposite of the outlet opening, or the inlet opening may for example be positioned in a side wall of the circumferential member.

The central body may comprise a central shaft and one or more circumferential support protrusions protruding from the central shaft. At least one of the circumferential protrusions may be tapered towards the central shaft.

### BRIEF DESCRIPTION OF THE FIGURES

In the figures,
Figs. 1 and 2 schematically depict in a section view a first example of a device for use in a process for production of cultured meat;
Fig. 3 shows another embodiment of the first example of the device;
Fig. 4 shows yet another embodiment of the first example of the device;
Figs. 5A-5D schematically depict a cross-section of a second example of a device for use in a process for production of cultured meat;
Figs. 6A and 6B schematically depict a cross-section of another embodiment of the second example of the device; and
Figs. 6C and 6D schematically depict two embodiments of the central body.

### DETAILED DESCRIPTION OF THE FIGURES

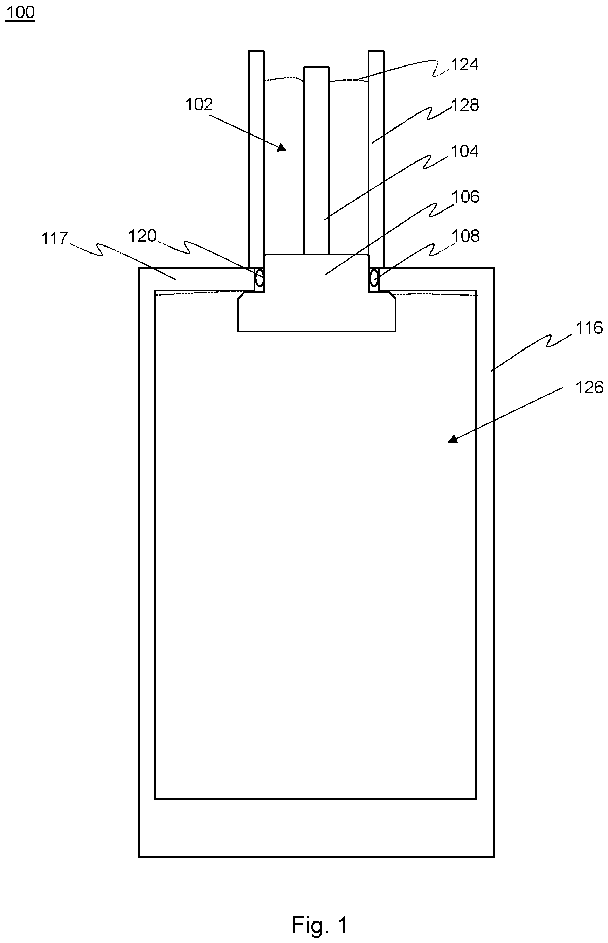

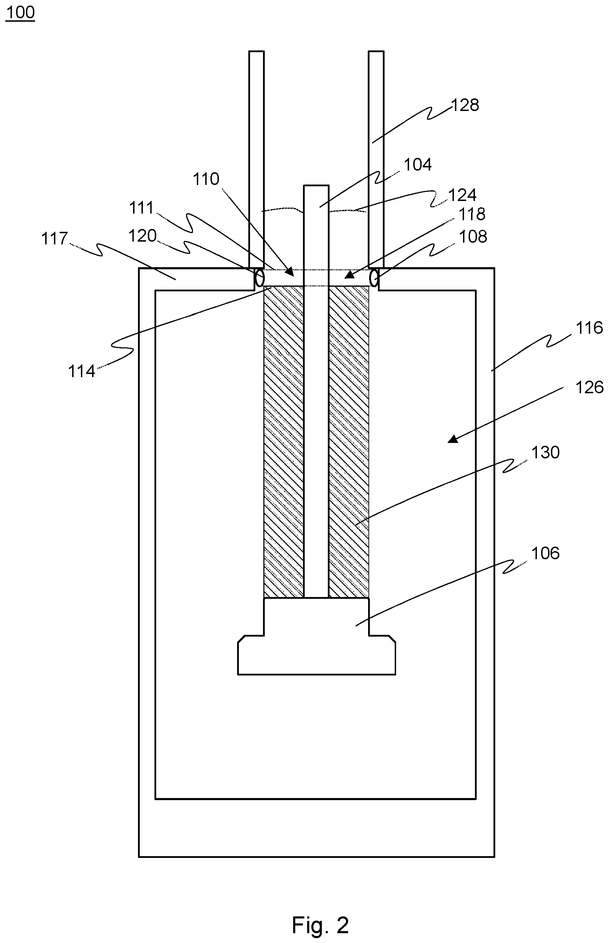

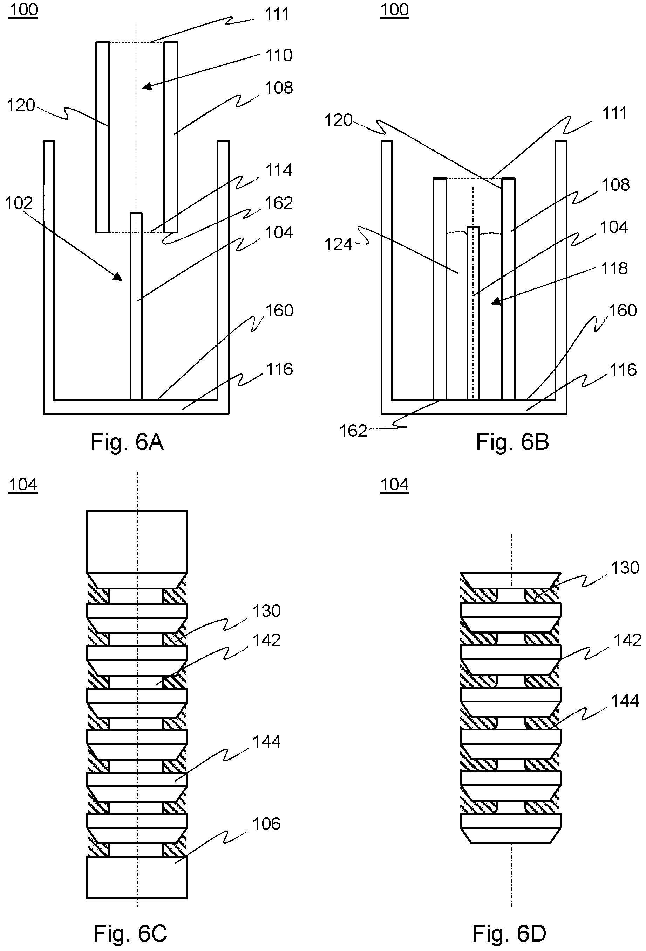

Figs. 1 and 2 schematically depict in a section view a first example of a device 100 for use in a process for production of cultured meat, in particular for preparing a cell-containing hydrogel, which device for example may be a bioreactor. The device 100 comprises a mould 102 comprising a central body 104 and a circumferential member 108. In general, the central body 104 may have a substantially constant cross-section shape. In other embodiments, the central body 104 may comprise sections with different cross-sectional shapes, such as a thickened sealing section 106. Examples of further central bodies 104 are elaborated on in conjunctions with Figs. 6C and 6D.

The central body 104 is positioned inside a cell-containing fluid container 128, holding a volume of cell-containing fluid 124. In the embodiment and situation of Fig. 1, the cell-containing fluid 124 is contained between the thickened sealing section 106 and the cell-containing fluid container 128. The cell-containing fluid container 128 may for example by embodied as a hollow cylinder, or any other hollow shaped member. The cell-containing fluid container 128 may as an option be formed by the circumferential member 108. The central body 104 may have a greater height in its elongation direction than a height of the circumferential member 108.

The device 100 further comprises a cross-linking fluid container 116 with a storage volume for holding a cross-linking fluid 126. Preferably, the storage volume is essentially fully filled with the cross-linking fluid 126, to prevent or reduce presence of a headspace into which cell-containing fluid may leak before being cross-linked into cell-containing hydrogel. Essentially fully filled may be understood for example as more than 95% filled, or even more than 99% filled. In other terms, essentially fully filled may imply that a headspace above the cross-linking fluid 126 is smaller than 5 cm, smaller than 2 cm, or even smaller than 1 cm. The cross-linking fluid container 116 may be provided with a lid 117 for containing the cross-linking fluid 126.

Fig. 1 shows the device 100 in a sealing position in which the central body 104, and in particular the thickened sealing section 106, forms an essentially liquid-tight seal with an inner wall 120 of the circumferential member 108. Fig. 2 shows the device 100 in a non-sealing position, in which part of the central body 104, is not surrounded by the circumferential member 108. In the sealing position, the cell-containing fluid 124 may substantially not leak into the cross-linking fluid container 116, although in use some leakage may occur.

For example when the device 100 is in the sealing position, at least part of the cell-containing fluid container 128 and/or the hollow passage 110 may be filled with cell-containing fluid 124.

To transition between the sealing position and the non-sealing position, the central body 104 is moved relative to the circumferential member 108. In particular, the central body 104 may be moved downward into the cross-linking fluid container, using any actuator, for example an electric, hydraulic, or pneumatic actuator (not shown in the figures).

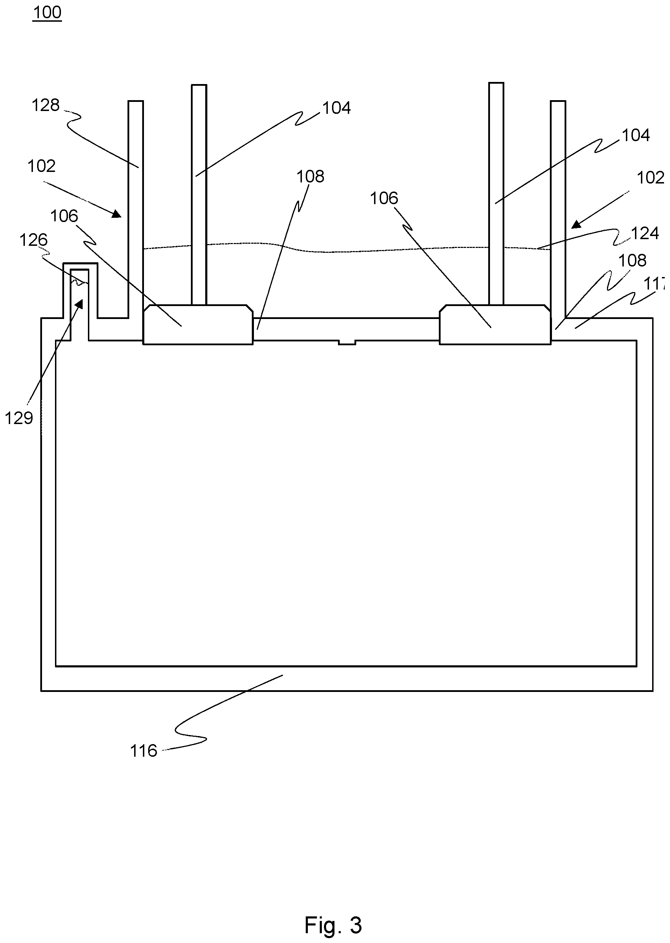

Fig. 3 shows an embodiment of the device 100 comprising more than one central body 104. To increase production capacity of the device 100, a device 100 such as a bioreactor may comprise any number of central bodies 104, for example one, two, ten or more, one hundred or more, or even five hundred or more. The actuator for moving one central body may be arranged for simultaneously moving multiple central bodies. For example, multiple central bodies may be connected to each other to couple their movements.

As a further option shown in Fig. 3, multiple central bodies 104 may be positioned in a single cell-containing fluid container 128, and as such from a single source of cell-containing fluid, multiple circumferential members 108 may be filled. The cell-containing fluid container 128 may be formed by part of the cross-linking fluid container 116. At least part of one or more circumferential members may be formed by part of the cross-linking fluid container 116.

It will be understood that any option disclosed in conjunction with any of the embodiments of Figs. 1-4 may be readily applied to other embodiments of the first or second example of the device, in particular to the embodiments of another one of the Figs. 1-4.

The circumferential member 108 has a hollow passage 110 therethrough, indicated in Fig. 2. The hollow passage 110 is provided with an inlet opening 111 and an outlet opening 114, for example on an opposite side of the hollow passage 110. The outlet opening 114 may be arranged for allowing passage of at least part of the central body 104.

In use, for example while the central body 104 is lowered in to the cross-linking fluid container 116, part of the hollow passage 110 may be filled with cell-containing fluid 124. As shown in Fig. 2, part of the cell-containing fluid 124 may have already been gelated into cell-containing hydrogel 130, whilst another part of the cell-containing fluid 124 is still present in the hollow passage 110 in a more fluid form.

As a particular option, the circumferential member 108 may be formed or comprised by the cross-linking fluid container 116, and/or by the cell-containing fluid container 128. As a general option, any circumferential member 108 may comprise a plurality of hollow passages.

The circumferential member 108 is for example in the embodiment of Fig. 2 connected to the cross-linking fluid container 116. The cross-linking fluid container 116, in particular the lid 117, comprises an opening which is substantially aligned with the hollow passage 110 of the circumferential member 108 and allows passage of the central body 104 and at least part of the optional thickened sealing section 106 into the cross-linking fluid container 116.

As the central body 104 is moved into the cross-linking fluid container 116, the cell-containing fluid 124 is put into contact with the cross-linking fluid 126, which in particular is a fast cross-linking fluid. The contact causes the cell-containing fluid 124 to cross-link into a cell-containing hydrogel 130, shown hatched in Fig. 2.

The particular shape of the cell-containing hydrogel 130 is defined by an annular volume 118 between the central body 104 and an inner wall 120 of the circumferential member 106 - because the cross-linking process is sufficiently fast.

An optional overflow buffer may be comprised by the device, for example the devices 100 of Figs. 1, 2 and 3. As the central body 104 or central bodies 104 are moved into the cross-linking fluid container 116, part of the storage volume therein becomes occupied with the central bodies 104 and the cell-containing fluid. The overflow buffer allows the volume in which cross-linking fluid can be present to be dynamic. A control unit may be present for controlling the volume of cross-linking fluid inside the cross-linking fluid container 116, for example with an aim to keep the cross-linking fluid container 116 essentially fully filled. The control unit may for example be arranged to control one or more pumps and/or valves. The overflow buffer may at least partially be positioned above the lid 117.

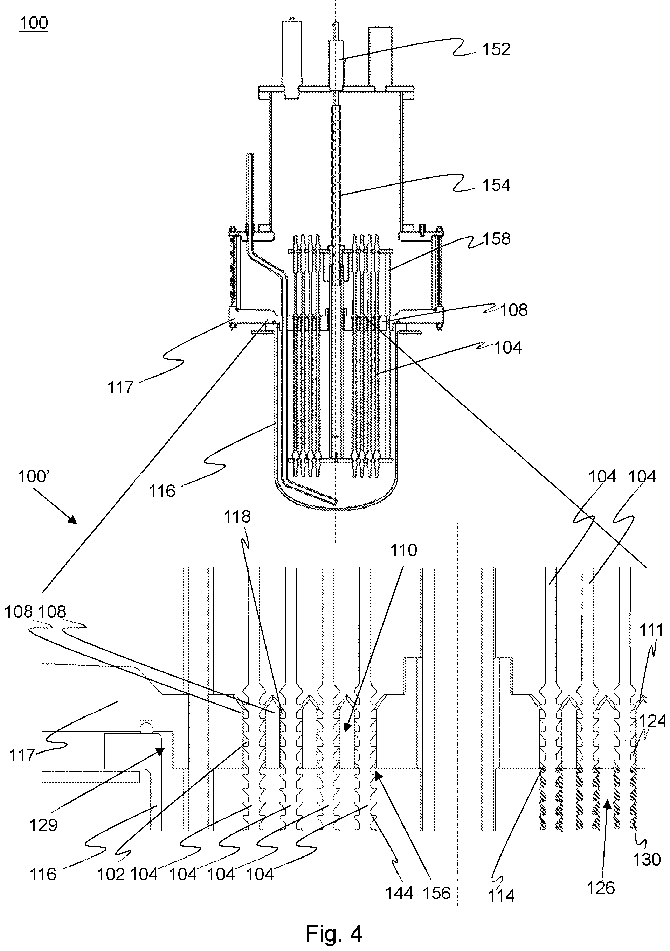

An example of an overflow buffer 129 is indicated in Figs. 3 and 4. It may be an object of a control unit to maintain a volume of cross-linking fluid in the overflow buffer 129, and the overflow buffer 129 may be positioned above an access area between the central body and the circumferential member where the cross-linking fluid 126 contacts the cell-containing fluid 124.

Fig. 4 schematically shows a section view of another embodiment of the first example of the device 100. In particular, Fig. 4 shows the cross-section and a detail 100' thereof.

The embodiment of the device 100 depicted in Fig. 4 comprises a plurality of central bodies 104, which are connected to and moveable by an actuator 152, for example by a spindle 154. The actuator 152 may be an electric motor. By operating the actuator 152, the central bodies 104 may be moved relative to the cross-linking fluid container 116, and the circumferential member 108. For example using a connection frame 158, bottom parts and top parts of the central bodies 104 may be connected.

The circumferential member 108 is formed by a lid 117 of the container, and comprises a plurality of through-holes as hollow passages. One hollow passage may be provided per central body 104.

In the detailed view of the device 100', the plurality of central bodies 104 is shown protruding into hollow passages 110 formed by the circumferential member 108. Per central body 104, one or more annular volume 102 are formed with the circumferential member 108. An access area 156 is provided between the central body 104 and the circumferential member 108 where cell-containing fluid 124 can be cross-linked into cell-containing hydrogel 130.

It will be understood that not all components of the device 100 in Fig. 4 have been provided with a reference numeral for conciseness and clarity of the figure. Only some central bodies have been depicted with the cell-containing fluid 124 and cell-containing hydrogel 130 (hatched).

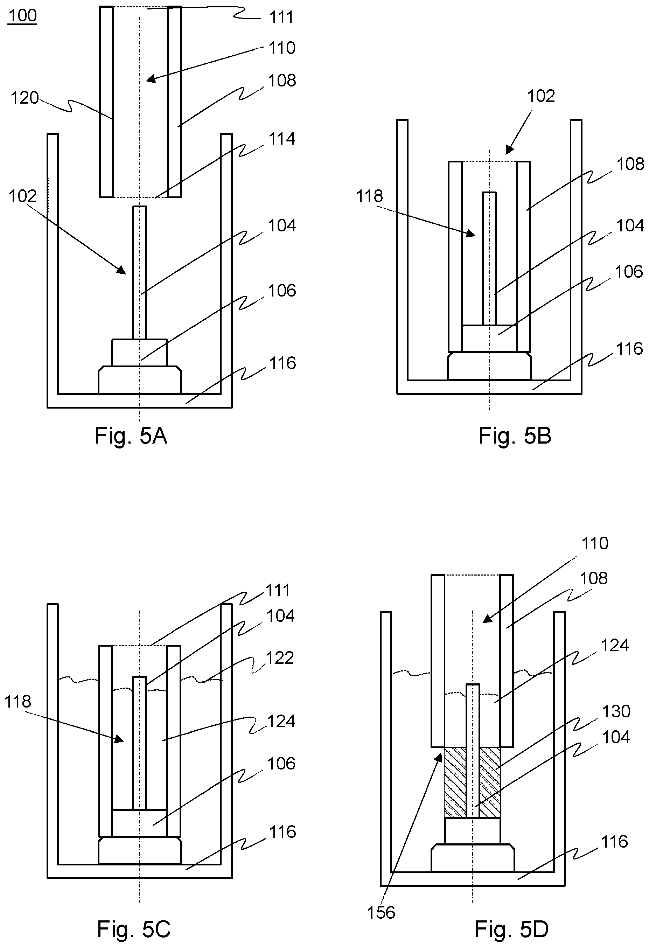

Figs. 5A-5D schematically depict a cross-section of second example of a device 100 for use in a process for production of cultured meat, in particular for preparing a cell-containing hydrogel. The device 100 comprises a cross-linking fluid container 116 and a central body 104 positioned inside the cross-linking fluid container 116, and for example connected to the cross-linking fluid container 116.

In Figs. 5B and 5C, the circumferential member 108 is depicted in a sealing position in which an essentially liquid-tight seal is formed with the circumferential member 108 and in this example a thickened sealing section 106 of the central body 104. Alternatively, as depicted in Fig. 6B, an essentially liquid-tight seal may be obtained between the circumferential member 108 and the cross-linking fluid container 116.

Between Figs. 5A-5D, the following method steps are performed. In the situation of Fig. 5A, the circumferential member 108 is positioned above the central body 104, for example using any actuator arranged to move the circumferential member 108 relative to the central body 104 and/or the cross-linking fluid container 116.

In the situation of Fig. 5B, the circumferential member 108 has been lowered over the central body 104, and as such an annular volume 118 is formed for receiving a cell-containing fluid in. An essentially liquid-tight seal is formed between the circumferential member 108 and the central body 104, in particular the thickened sealing section 106. Here and in general, essentially liquid-tight implies that sufficient cell-containing fluid can be contained in order to form the cell-containing hydrogel. Some leakage may be acceptable.

In the transition between the situation of Fig. 5A and the situation of Fig. 5B, the central body 104 passes into the circumferential member 108 via the outlet opening 114. Also during the transition between the situation of Fig. 6A and the situation of Fig. 6B, the central body 104 passes into the circumferential member 108 via the outlet opening 114.

From the first example and the second example of the device 100, it will be understood that moving the central body and the circumferential member relative to each other may imply a movement of the central body, a movement of the circumferential member, or a movement of both.

In the situation of Fig. 5C, the annular volume 118 has been filled with cell-containing fluid 124, and outside of the circumferential member 108, the cross-linking fluid container 116 has been filled with the cross-linking fluid 122. The cell-container fluid 124 is supplied into the annular volume via the inlet opening 111.

In the situation of Fig. 5D, the circumferential member 108 has been lifted up and away from the central body 104 and from a bottom of the cross-linking fluid container 116, but still partially surrounds an upper part of the central body 104. As schematically depicted in Fig. 5D, part of the cell-containing fluid 124 has been cross-linked into cell-containing hydrogel 130 (shown hatched). Because the cross-linking process is sufficiently fast, the shape of the cell-containing fluid 124 has been determined by the shape of the central body 104 and hollow passage 110 of the circumferential member 108, which central body 104 and circumferential member 108 thus form a mould 102. The circumferential member 108 may be lifted further up and away from the central body 104.

Figs. 6A and 6B schematically depict a cross-section of another embodiment of the second example of the device 100 for use in a process for production of cultured meat. In this particular embodiment, the central body 104 protrudes away from the cross-linking fluid container 116. More in particular, the central body 104 protrudes upwards from a bottom 160 if the fluid container 116. In general, the central body 104 may be connectable to the bottom, or may be even be integrally formed with the fluid container 116. The bottom 160 may have a substantially flat surface relative to which the central body 104 protrudes.

Compared to Fig. 6A, in the situation depicted in Fig. 6B the central body 104 has been moved relative to the circumferential member 108. By virtue of this movement, the circumferential member 108 has been moved relative to the bottom 160 of the fluid container 116. In the situation of Fig. 6B, a lower end face 162 of the circumferential member 108 facing the bottom 160 forms a substantially liquid-tight seal with the fluid container 116, in particular with the bottom 160 of the fluid container 116. By virtue of this seal, the annular volume 118 between the central body and the circumferential member can be filled with cell-containing fluid 124 and the cell-containing fluid 124 can be held in said annular volume. For example during or after filling the annular volume 118 with cell-containing fluid 124, the fluid container 116 may be filled with cross-linking fluid. By subsequently moving the circumferential member 108 away relative to the bottom 160 of the fluid container 116, the cell-containing fluid is exposed to the cross-linking fluid.

Optionally, not depicted in the figures, one or more sealing members may be positioned between the lower end face 162 of the circumferential member 108 and the bottom 160 of the fluid container 116 to provide or improve the essentially liquid-tight seal. Such a sealing member may for example be a resilient sealing member.

Fig. 6C and 6D schematically depicts two embodiments of the central body 104. The embodiments may be readily used in any embodiment of the device 100, in particular the devices 100 depicted in any of the Figs. 1A-6B. The central bodies 104 shown in Figs. 6C and 6D comprise a central shaft 142, and a plurality of circumferential support protrusions 144. After being used in for example the first example or the second example of the device 100, cell-containing hydrogel 130 may be present between the circumferential support protrusions 144, as shown in Fig. 6C.

As a particular option, at least part of one or more of the circumferential support protrusions 144 may be tapered, in particular tapered towards the thickened sealing section 106 or in use in a direction of gravity. The support protrusions 144 may be used for preventing cell-containing hydrogel from collapsing of the central body 104, for example by the weight of the cell-containing hydrogel pressing down on itself.

In the embodiment of Fig. 6D, an option for any central body 104 is depicted. Whereas the central shaft 142 in Fig. 6C is shown with an essentially constant cross-sectional shape, in embodiments, the central body 104 may have a non-constant cross-sectional shape. For example, a transition between the central shaft 142 and a circumferential support protrusion 144 may be rounded, provided with a radius, and/or bevelled.

As can be seen from Fig. 6C, and outer shape of the thickened sealing section 106 may correspond or approximately corresponds to an outer shape of the support protrusions 144.

The central shaft 142 may have a generally circular cross-sectional shape, or any other shape such as an oval, racetrack, rounded rectangle, or any other shape. The cross-section shape of a thickened sealing section 106 and/or a support protrusion 144 may correspond to the cross-section shape of the central shaft 142, albeit with different dimensions.

In summary, a method of preparing a cell-containing hydrogel for use in the production of cultured meat is provided, along with examples of device for performing the method. The method comprises steps of positioning a central body inside a circumferential member, thereby forming an annular volume, filling the annular volume with a cell-containing fluid, moving the central body relative to the circumferential member such that the central body moves through an outlet opening of the circumferential member, thereby creating an access area between the central body and the circumferential member, and exposing the access area to a cross-linking fluid to allow crosslinking of the cell-containing fluid to obtain a cell-containing hydrogel.

In the description above, it will be understood that when an element is referred to as being connect to another element, the element is either directly connected to the other element, or intervening elements may also be present. Also, it will be understood that the values given in the description above, are given by way of example and that other values may be possible and/or may be strived for.

## Claims

1. Method of preparing a cell-containing hydrogel for use in the production of cultured meat, the method comprising the steps of:
- positioning a central body inside a circumferential member, thereby forming an annular volume (118) between the central body and an inner wall (120) of the circumferential member;
- filling the annular volume with a cell-containing fluid (124),
- moving the central body relative to the circumferential member such that the central body moves through an outlet opening (114) of the circumferential member, thereby creating an access area (156) between the central body and the circumferential member; and
- exposing the access area to a cross-linking fluid (126) to allow crosslinking of the cell-containing fluid to obtain a cell-containing hydrogel.

2. Method according to claim 1, further comprising, prior to or during the filling of at least part of the annular volume with the cell-containing fluid, moving the circumferential member over the central body.

3. Method according to claim 1 or 2, further comprising, after or during the filling of at least part of the annular volume with the cell-containing fluid, moving the central body through the outlet opening of the circumferential member.

4. Method according to any of the preceding claims, wherein the cell-containing fluid comprises a polysaccharide, preferably wherein the cell-containing fluid comprises alginate.

5. Method according to claim 4, wherein the polysaccharide is conjugated with one or more cell-adhesion peptides.

6. Method according to any of the preceding claims, wherein the cross-linking fluid comprises a divalent cation, preferably calcium (Ca²⁺) cations, a covalent crosslinker and/or wherein the cross-linking fluid comprises a buffer.

7. Method according to any of the preceding claims, wherein the cell-containing fluid is at least partially crosslinked by a forming fluid before exposing the cell-containing fluid to the crosslinking fluid.

8. Method of producing cultured meat, comprising the steps of:
- obtaining a cell-containing hydrogel using a method according to any of the claims 1-7;
- incubating the cell-containing hydrogel in a differentiation medium to form tissue; and
- producing the cultured meat from the formed tissue.

9. Device (100) for use in a process for production of cultured meat, the device comprising:
- a mould (102), comprising:
- a central body (104); and
- a circumferential member (108) with a hollow passage (110) therethrough, which hollow passage is provided with an inlet opening (111) for receiving a cell-containing fluid in the hollow passage, and an outlet opening (114);
- a cross-linking fluid container (116) with a storage volume for holding a cross-linking fluid, wherein the cross-linking fluid container is arranged for accommodating at least part of the central member;
wherein
- the central body is arranged to be positioned at least partially inside the hollow passage of the circumferential member, thereby forming an annular volume (118) between the central body and an inner wall (120) of the circumferential member;
- the outlet opening is arranged to allow passage of at least part of the central body; and
- the central body and the circumferential member are arranged to be moved relative to each other in a movement in which at least part of the central body passes through the outlet opening.

10. Device according to claim 9, wherein the central body is positioned in the storage volume of the cross-linking fluid container, and the circumferential member is arranged to be moved between:
- a non-sealing position in which at least part of the central body is not surrounded by the circumferential member; and
- a sealing position in which an essentially liquid-tight seal is formed with the circumferential member for holding a cell-containing fluid in the annular volume (118) between the central body and the inner wall (120) of the circumferential member.

11. Device according to claim 9 or 10, further comprising an actuator for moving the circumferential member relative to the central body in a direction generally parallel to an elongation direction of the central body.

12. Device according to any of the claims 9-11, wherein the circumferential member is fixated relative to the cross-linking fluid container, and the central body is arranged to be at least partially moved into the cross-linking fluid container through the hollow passage and the outlet opening of the circumferential member.

13. Device according to any of the claims 9-12, further comprising a cell-containing fluid container for holding a volume of cell-containing fluid, and a pump for transporting cell-containing fluid into the hollow passage of the circumferential member via the inlet opening.

14. Device according to any of the preceding claims, wherein the central body comprises a central shaft (142) and one or more circumferential support protrusions (144) protruding from the central shaft.

15. Device according to claim 14, wherein at least one of the circumferential support protrusions is tapered towards the central shaft.

## Patentansprüche

1. Verfahren zur Herstellung eines zellhaltigen Hydrogels zur Verwendung bei der Produktion von kultiviertem Fleisch, wobei das Verfahren die Schritte umfasst:
- Positionieren eines zentralen Körpers innerhalb eines Umfangselements, wodurch ein ringförmiges Volumen (118) zwischen dem zentralen Körper und einer Innenwand (120) des Umfangselements gebildet wird;
- Füllen des ringförmigen Volumens mit einer zellhaltigen Flüssigkeit (124),
- Bewegen des zentralen Körpers relativ zum Umfangselement, so dass der zentrale Körper sich durch eine Auslassöffnung (114) des Umfangselements bewegt, wodurch ein Zugangsbereich (156) zwischen dem zentralen Körper und dem Umfangselement gebildet wird; und
- Aussetzen des Zugangsbereichs einer Vernetzungsflüssigkeit (126), um eine Vernetzung der zellhaltigen Flüssigkeit zu ermöglichen und ein zellhaltiges Hydrogel zu erhalten.

2. Verfahren nach Anspruch 1, das ferner vor oder während des Füllens von zumindest einem Teil des ringförmigen Volumens mit der zellhaltigen Flüssigkeit das Bewegen des Umfangselements über den zentralen Körper umfasst.

3. Verfahren nach Anspruch 1 oder 2, das weiterhin umfasst, dass nach oder während des Füllens von zumindest einem Teil des ringförmigen Volumens mit der zellhaltigen Flüssigkeit der Zentralkörper durch die Auslassöffnung des Umfangselements bewegt wird.

4. Verfahren nach einem der vorhergehenden Ansprüche, wobei die zellhaltige Flüssigkeit ein Polysaccharid umfasst, vorzugsweise wobei die zellhaltige Flüssigkeit Alginat umfasst.

5. Verfahren nach Anspruch 4, wobei das Polysaccharid mit einem oder mehreren Zelladhäsionspeptiden konjugiert ist.

6. Verfahren nach einem der vorhergehenden Ansprüche, wobei die Vernetzungsflüssigkeit ein zweiwertiges Kation, vorzugsweise Calciumkationen (Ca 2+ ), einen kovalenten Vernetzer umfasst und/oder wobei die Vernetzungsflüssigkeit einen Puffer umfasst.

7. Verfahren nach einem der vorhergehenden Ansprüche, wobei die zellhaltige Flüssigkeit zumindest teilweise durch eine Formflüssigkeit vernetzt wird, bevor die zellhaltige Flüssigkeit der Vernetzungsflüssigkeit ausgesetzt wird.

8. Verfahren zur Herstellung von kultiviertem Fleisch, umfassend die Schritte:
- Erhalten eines zellhaltigen Hydrogels unter Verwendung eines Verfahrens gemäß einem der Ansprüche 1 bis 7;
- Inkubieren des zellhaltigen Hydrogels in einem Differenzierungsmedium zur Bildung von Gewebe; und
- Herstellen des kultivierten Fleisches aus dem gebildeten Gewebe.

9. Vorrichtung (100) zur Verwendung in einem Verfahren zur Herstellung von kultiviertem Fleisch, wobei die Vorrichtung umfasst:
- eine Form (102), umfassend:
- einen zentralen Körper (104); und
- ein Umfangselement (108) mit einem hohlen Durchgang (110), der mit einer Einlassöffnung (111) zum Aufnehmen einer zellhaltigen Flüssigkeit in dem hohlen Durchgang und einer Auslassöffnung (114) versehen ist;
- einen Vernetzungsflüssigkeitsbehälter (116) mit einem Speichervolumen zum Aufnehmen einer Vernetzungsflüssigkeit, wobei der Vernetzungsflüssigkeitsbehälter so angeordnet ist, dass er zumindest einen Teil des zentralen Elements aufnehmen kann;
wobei
- der Zentralkörper so angeordnet ist, dass er zumindest teilweise innerhalb des hohlen Durchgangs des Umfangselements positioniert ist, wodurch ein ringförmiges Volumen (118) zwischen dem Zentralkörper und einer Innenwand (120) des Umfangselements gebildet wird;
- die Auslassöffnung so angeordnet ist, dass sie den Durchgang von zumindest einem Teil des Zentralkörpers ermöglicht; und
- der Zentralkörper und das Umfangselement so angeordnet sind, dass sie in einer Bewegung relativ zueinander bewegt werden, bei der zumindest ein Teil des Zentralkörpers durch die Auslassöffnung hindurchtritt.

10. Vorrichtung nach Anspruch 9, wobei der Zentralkörper im Speichervolumen des Behälters für die Vernetzungsflüssigkeit positioniert ist und das Umfangselement so angeordnet ist, dass es zwischen folgenden Positionen bewegt werden kann:
- einer nicht abdichtenden Position, in der zumindest ein Teil des Zentralkörpers nicht von dem Umfangselement umgeben ist; und
- einer abdichtenden Position, in der eine im Wesentlichen flüssigkeitsdichte Abdichtung mit dem Umfangselement gebildet wird, um eine zellenhaltige Flüssigkeit in dem ringförmigen Volumen (118) zwischen dem Zentralkörper und der Innenwand (120) des Umfangselements zu halten.

11. Vorrichtung nach Anspruch 9 oder 10, die außerdem einen Aktuator zum Bewegen des Umfangselements relativ zum Zentralkörper in eine Richtung umfasst, die im Allgemeinen parallel zu einer Ausdehnungsrichtung des Zentralkörpers ist.

12. Vorrichtung nach einem der Ansprüche 9-11, wobei das Umfangselement relativ zum Behälter für die Vernetzungsflüssigkeit fixiert ist und der Zentralkörper so angeordnet ist, dass er zumindest teilweise durch den Hohlkanal und die Auslassöffnung des Umfangselements in den Behälter für die Vernetzungsflüssigkeit bewegt werden kann.

13. Vorrichtung nach einem der Ansprüche 9-12, die außerdem einen Behälter für zellhaltige Flüssigkeit zum Aufnehmen eines Volumens zellhaltiger Flüssigkeit und eine Pumpe zum Transportieren zellhaltiger Flüssigkeit in den Hohlkanal des Umfangselements über die Einlassöffnung umfasst.

14. Vorrichtung nach einem der vorhergehenden Ansprüche, wobei der Zentralkörper eine Zentralwelle (142) und einen oder mehrere Umfangsstützvorsprünge (144) umfasst, die von der Zentralwelle vorstehen.

15. Vorrichtung nach Anspruch 14, wobei mindestens einer der Umfangsstützvorsprünge in Richtung der Zentralwelle verjüngt ist.

## Revendications

1. Procédé de préparation d'un hydrogel contenant des cellules destiné à être utilisé dans la production de viande cultivée, le procédé comprenant les étapes consistant à :
- positionner un corps central à l'intérieur d'un élément circonférentiel, formant ainsi un volume annulaire (118) entre le corps central et une paroi interne (120) de l'élément circonférentiel;
- remplir le volume annulaire avec un fluide contenant des cellules (124),
- déplacer le corps central par rapport à l'élément circonférentiel de telle sorte que le corps central se déplace à travers une ouverture de sortie (114) de l'élément circonférentiel, créant ainsi une zone d'accès (156) entre le corps central et l'élément circonférentiel ; et
- exposer la zone d'accès à un fluide de réticulation (126) pour permettre la réticulation du fluide contenant des cellules afin d'obtenir un hydrogel contenant des cellules.

2. Procédé selon la revendication 1, comprenant en outre, avant ou pendant le remplissage d'au moins une partie du volume annulaire avec le fluide contenant des cellules, le déplacement de l'élément circonférentiel sur le corps central.

3. Procédé selon la revendication 1 ou 2, comprenant en outre, après ou pendant le remplissage d'au moins une partie du volume annulaire avec le fluide contenant des cellules, le déplacement du corps central à travers l'ouverture de sortie de l'élément circonférentiel.

4. Procédé selon l'une quelconque des revendications précédentes, dans lequel le fluide contenant des cellules comprend un polysaccharide, de préférence dans lequel le fluide contenant des cellules comprend de l'alginate.

5. Procédé selon la revendication 4, dans lequel le polysaccharide est conjugué à un ou plusieurs peptides d'adhésion cellulaire.

6. Procédé selon l'une quelconque des revendications précédentes, dans lequel le fluide de réticulation comprend un cation divalent, de préférence des cations calcium (Ca 2+ ), un agent de réticulation covalent et/ou dans lequel le fluide de réticulation comprend un tampon.

7. Procédé selon l'une quelconque des revendications précédentes, dans lequel le fluide contenant des cellules est au moins partiellement réticulé par un fluide de formation avant d'exposer le fluide contenant des cellules au fluide de réticulation.

8. Procédé de production de viande cultivée, comprenant les étapes consistant à :
- obtenir un hydrogel contenant des cellules en utilisant un procédé selon l'une quelconque des revendications 1 à 7 ;
- incuber l'hydrogel contenant des cellules dans un milieu de différenciation pour former un tissu ; et
- produire la viande cultivée à partir du tissu formé.

9. Dispositif (100) destiné à être utilisé dans un procédé de production de viande cultivée, le dispositif comprenant :
- un moule (102), comprenant :
- un corps central (104) ; et
- un élément circonférentiel (108) avec un passage creux (110) à travers celui-ci, lequel passage creux est pourvu d'une ouverture d'entrée (111) pour recevoir un fluide contenant des cellules dans le passage creux, et d'une ouverture de sortie (114) ;
- un récipient de fluide de réticulation (116) avec un volume de stockage pour contenir un fluide de réticulation, le récipient de fluide de réticulation étant conçu pour recevoir au moins une partie de l'élément central ;
dans lequel
- le corps central est agencé pour être positionné au moins partiellement à l'intérieur du passage creux de l'élément circonférentiel, formant ainsi un volume annulaire (118) entre le corps central et une paroi interne (120) de l'élément circonférentiel ;
- l'ouverture de sortie est agencée pour permettre le passage d'au moins une partie du corps central ; et
- le corps central et l'élément circonférentiel sont agencés pour être déplacés l'un par rapport à l'autre dans un mouvement dans lequel au moins une partie du corps central passe à travers l'ouverture de sortie.

10. Dispositif selon la revendication 9, dans lequel le corps central est positionné dans le volume de stockage du récipient de fluide de réticulation, et l'élément circonférentiel est agencé pour être déplacé entre :
- une position de non-étanchéité dans laquelle au moins une partie du corps central n'est pas entourée par l'élément circonférentiel ; et
- une position d'étanchéité dans laquelle un joint essentiellement étanche aux liquides est formé avec l'élément circonférentiel pour maintenir un fluide contenant des cellules dans le volume annulaire (118) entre le corps central et la paroi interne (120) de l'élément circonférentiel.

11. Dispositif selon la revendication 9 ou 10, comprenant en outre un actionneur pour déplacer l'élément circonférentiel par rapport au corps central dans une direction généralement parallèle à une direction d'allongement du corps central.

12. Dispositif selon l'une quelconque des revendications 9 à 11, dans lequel l'élément circonférentiel est fixé par rapport au récipient de fluide de réticulation, et le corps central est agencé pour être au moins partiellement déplacé dans le récipient de fluide de réticulation à travers le passage creux et l'ouverture de sortie de l'élément circonférentiel.

13. Dispositif selon l'une quelconque des revendications 9 à 12, comprenant en outre un récipient de fluide contenant des cellules pour contenir un volume de fluide contenant des cellules, et une pompe pour transporter le fluide contenant des cellules dans le passage creux de l'élément circonférentiel via l'ouverture d'entrée.

14. Dispositif selon l'une quelconque des revendications précédentes, dans lequel le corps central comprend un arbre central (142) et une ou plusieurs saillies de support circonférentielles (144) faisant saillie à partir de l'arbre central.

15. Dispositif selon la revendication 14, dans lequel au moins une des saillies de support circonférentiel est conique vers l'arbre central.
